# EUROPEAN PATENT SPECIFICATION

(11) **EP 1 535 634 B1**
(45) Date of publication and mention of the grant of the patent: **29.08.2012**
(21) Application number: 03425762.6
(22) Date of filing: 28.11.2003
(51) Int. Cl.: A61M 1/00

(54) **Container for picking up organic liquids**
Behälter zur Aufnahme von organischen Fluiden
Réservoir pour recueillir des fluides organiques

(43) Date of publication of application: 01.06.2005
(73) Proprietor: FLOW-METER S.p.A., I-24020 Levate Bergamo (IT)
(72) Inventor: Paratico, Roberto c/o Flow-Meter S.p.a., 24040 Levate (IT)
(74) Representative: Botti, Mario

(56) References cited:
- EP-A- 0 358 302
- US-A- 3 646 935
- US-A- 3 955 572
- US-A- 4 013 076
- US-A- 5 667 485
- US-A- 5 960 837

## Description

### Field of application

The present invention refers to a container for picking up organic liquids sucked from patients and/or invalid people. The container comprises a beaker and a lid hermetically closed on the mouth of the beaker itself. The lid is provided with connecting elements for connection, on one side, with a vacuum source and, on the other, to a duct for liquid suction.

### Prior Art

Containers for picking up organic liquids sucked from patients and/or invalid people are known. These containers need to be hermetically closed to enable suction of the organic liquids and to preserve absolute hygiene outside.

In particular, a completely "disposable" container is known, which is substantially composed of a beaker with its own mouth closed on top by a lid. This latter is provided, on its external surface, with a first connecting element which is in communication with the inside of the beaker and is suitable to be connected to a vacuum source, and with a second connecting element, also in communication with the inside of the beaker, which is suitable to be connected to a sucking duct associated to the patient for picking up liquids.

When the vacuum source is activated, the container is depressed and the organic liquids are sucked and picked up directly in the beaker. Normally, once the operation has been completed, the container is temporarily disconnected from the sucking duct and it is kept connected to the vacuum source, thus remaining under high depression conditions. Later, when the beaker is full, the entire container is discarded.

In particular, in order to guarantee effective suction in the beaker and to preserve the hygienic conditions of the entire container, the lid is firmly fixed to the beaker itself and it is hermetically closed with it.

Prior art patents US 5, 960,837 and US 4,013,076 provides containers wherein on the internal surface of said lid a peripheral seat is formed for an elastic insertion of a corresponding terminal section of the mouth of the beaker. Mutual coupling means are provided to fasten said terminal section to said peripheral seat.

The disadvantage of this type of containers is the fact that it easily tends to crack or even break during use. This disadvantage is mainly due to the fact that the container is continuously connected to the vacuum source and it is therefore subjected to considerable mechanical stress. In general, the lid, together with the relative connecting elements, is the part of the container which is mainly subjected to these mechanical stress and which risks cracking or even breaking most frequently.

Furthermore, it should also be considered that this container, being of the disposable type, is manufactured, for reasons of cost-saving, in a plastic material with low mechanical properties, for example recycling material, and with low resistance to a high depression.

Therefore, the problem of the present invention is to manufacture a container for picking up and preserving organic liquids sucked from patients and/or invalid people, wherein the lid can be hermetically closed on the beaker and, at the same time, effectively withstand high mechanical stresses, even in the case it is manufactured in a material having low mechanical resistance properties.

### Summary of the invention

The technical problem is solved by a container as defined in the claims. A peripheral seat for elastic insertion of a terminal section of the mouth of the beaker is made on the internal surface of the lid. The container according to the invention also comprises mutual coupling means which fix the above terminal section of the mouth of the beaker into the peripheral seat.

The insertion into the peripheral seat and the contemporary coupling of the terminal section of the beaker ensure, on one hand, stability and fixing tightness and, on the other, thanks to the elastic configuration, they allow to discharge the mechanical stress to which mainly the lid is subjected.

For a better understanding of the characteristics and advantages of the container according to the present invention, a nonlimiting embodiment thereof is described below and is illustrated in the appended drawings.

### Brief description of the drawings

In said drawings:
Figure 1 is an axonometric top view of a first container according to the invention.
Figure 2 is an enlarged detail of a vertical section of the container of figure 1.
Figure 3 is an enlarged detail of the coupling system of the container in figure 2.
Figure 4 is an enlarged detail of a vertical section of a second container according to the invention.
Figure 5 is an enlarged detail of the insertion system of the container of figure 4.

### Detailed description

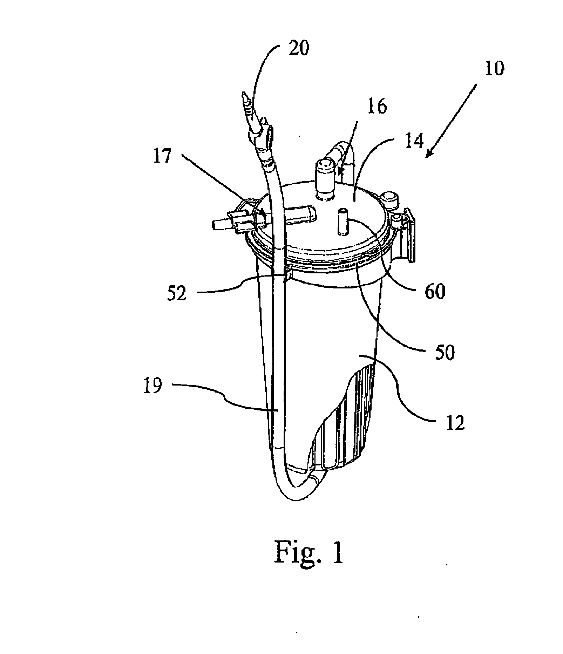

With reference to figure 1, a container 10 according to the present invention for picking up organic liquids sucked from patients and/or invalid people comprises a beaker12 and a lid 14, having a substantially circular shape, hermetically fixed at the mouth of the beaker 12 itself.

Both the lid 14 and the beaker 12 are advantageously manufactured by moulding a plastic material.

A first connecting element 16 and a second connecting element 17, of a substantially known type, are integrally formed on the flat external surface of the lid 14, these elements being suitable to be connected to a duct 19 for liquid suction and a vacuum source, not illustrated in the drawings, respectively. This latter is also provided with a terminal cannula 20.

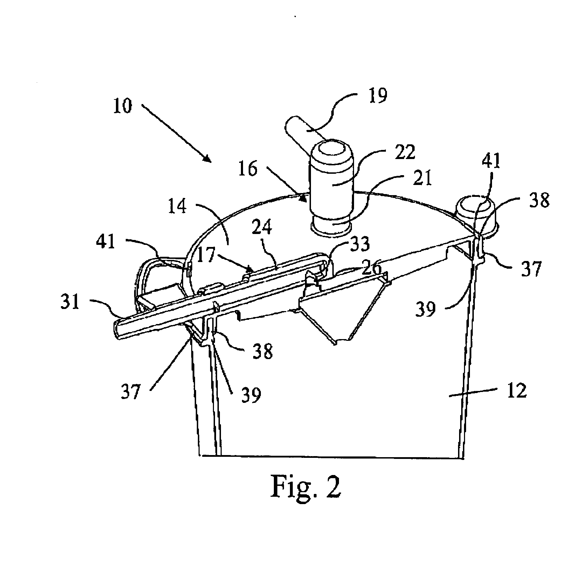

The first connecting element 16 has a vertical axis and it comprises a first sleeve 21 (figure 2), only partially visible in the drawings, which is suitable to be inserted into a second sleeve 22, having a greater diameter than the first and being connected to a terminal section of the suction duct 19.

The second connecting element 17 has a horizontal axis and it comprises a tubular portion 24 in communication with the inside of the beaker 12 through an opening 26 formed on the surface of the lid 14.

A connector 31, inserted in the tubular portion 24, is connected to the vacuum source and it is provided with a through hole 33. This latter is suitable to be aligned in correspondence with the opening 26, to create a depression in the beaker 12 when the vacuum source is activated.

When the container 10 is not in use, the connector 31 is turned around its axis to close the opening 26, thus interrupting suction inside the beaker 12. In this way, the container 10 itself does not have to be continually kept under pressure, thus preventing it from useless mechanical stress when suction of liquids is unnecessary.

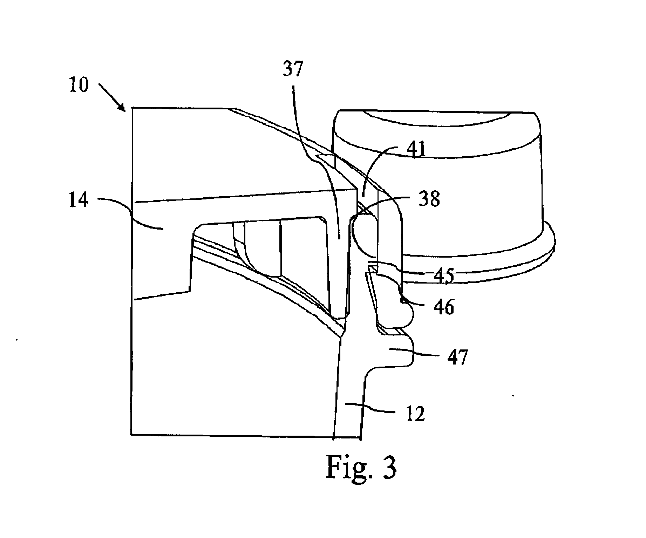

According to the present invention, in order to hermetically and firmly fasten the lid 14 to the beaker 12, a circular throat 38 (figure 3) is formed on the internal surface of the lid 14 itself, acting as a peripheral seat for the insertion of a corresponding terminal section 39 of the mouth of the beaker 12.

In particular, the circular throat 38, is defined inside a substantially vertical rim 37, integrally formed, which projects from the flat internal surface of the lid 14.

The particular configuration of the circular throat 38 formed inside the rim 37, enables elastic insertion of the terminal section 39. The elasticity of the insertion is also favoured by the plastic material with which the beaker 12 and the lid 14 are manufactured.

This characteristic of elasticity, besides facilitating insertion of the terminal section 39, also allows to obtain a coupling between the lid 14 and the cup 12 which effectively withstand the mechanical stress to which these elements are subjected when connected to the vacuum source.

The terminal section 39 is also provided with a first coupling tooth 45 which is suitable to co-operate with a second tooth 46 formed on the internal surface of the circular throat 38 in order to allow a firm and hermetic closing of the lid 14 and of the cup 12.

Advantageously, the tooth 45 is continuously formed throughout the surface of the terminal section 39, while the tooth 46 is discontinuously formed in the circular throat 38 in order to facilitate both the coupling to the tooth 45 by means of manual pressure and the spring-back of the plastic material.

In particular, the circular throat 38 comprises a plurality of teeth 46, angularly staggered with each other and, in correspondence of each tooth 46, the lid has a slit 41 (figures 2 and 3) to further facilitate elastic spring-back of the plastic material. The slit 41 also has the advantage of allowing the manufacture of the lid 14 by moulding.

According to another characteristic of the invention, a substantially horizontal circular fin 47, delimiting the lower part of the terminal section 39, is integrally formed on the outer surface of the beaker12.

In use, the circular fin 47 co-operates, by beating, with the lower side of the rim 37 to close the circular throat 38. The circular fin 47 has the advantage of preventing manual access to the terminal section 39 when this latter is inserted in the throat 38 itself, to uncouple the circular tooth 45.

Moreover, the circular fin 47 further guarantees hermetic tightness of the container 10 by means of pressure coupling on the internal wall of the rim 37.

In this way, a container 10 is obtained with the advantage of having a tight closure, being inviolable both intentionally and accidentally by a user, stable and resistant to mechanical stress and wherein absolute hygiene is preserved.

According to a further characteristic of the present invention, the container 10 is provided with a ring 50 (figure 1), wherein the beaker 12 is housed and which acts as a supporting element to fix the beaker 12 itself to wall.

In particular, the ring 50 can be removed from the beaker 12 and it can be re-used.

Furthermore, on the external wall of the ring 50, there is a retaining element 52 which allows to retain the duct 19 near container 10 to preventing obstruction. The retaining element 52 also has the advantage of keeping the duct 19 still when all the container 10 is subjected to suction, preventing accidental and uncontrollable movements.

According to a further characteristic of the present invention, the lid 14 is provided with a third connecting element 60, open towards the inside of the beaker 12, which allows to connect several containers 10 to each other in series, each provided with a relative suction duct 19 and of which only one is in communication with the vacuum source.

The connecting elements 60 of each container 10 can be connected to each other, for example, by means of a flexible tube not illustrated in the drawings.

The connection in series has the advantage of increasing the picking up capacity of the sucking system from several patients.

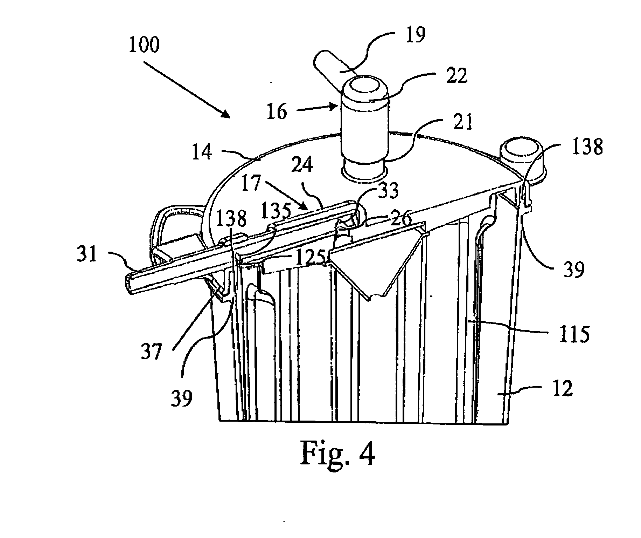

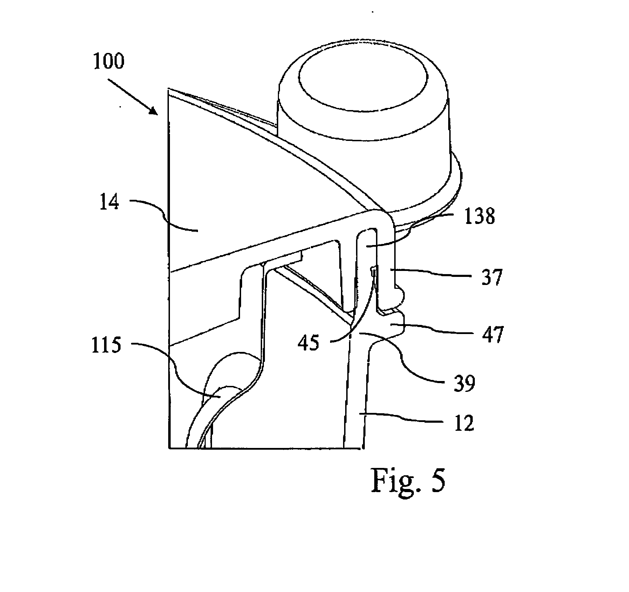

Figures 4 and 5 show a container 100 according to the invention, of the partially disposable type, where the beaker 12 can be re-used. The elements of the container 100 having the same characteristics and the same functions as already described above for the container 10, are indicated in figures 4 and 5 with the same reference numbers.

In particular, the container 100 is provided inside with a soft bag 115 which is directly welded on the internal surface of the lid 14, and is in communication with the first connecting element 16 and the second connecting element 17, in order to allow direct picking up of the liquid therein.

In this case, the beaker 12 simply acts as a supporting element for the soft bag 115.

The connector 31 has a second hole 135, which is aligned with a second opening 125 formed on the lid 14 to put the area between the inside of the beaker 12 and the outside of the soft bag 115 in communication with the vacuum source.

When the container 100 is connected to the vacuum source, thanks to the presence of the second hole 135 and the second opening 125, a balance of the suctionbetween the inside of the soft bag 115 and the inside of the beaker 12 is realised, allowing the soft bag 115 itself to stretch on the internal walls of the beaker 12.

In this way, the liquid is picked up directly in the soft bag 115. This latter and the lid 14, are eliminated after use while the beaker 12, which has no contacts with liquids, can be re-used.

Container 100, like container 10, is provided with the above-mentioned ring 50 for fixing to wall and with the retaining element 52 for the suction duct 19. The ring 50 and the retaining element 52 are not visible in figures 4 and 5.

According to the invention, the lid 14 is provided with a rim 37 (figure 5), inside of which a circular throat 138 is formed for the elastic insertion of the upper terminal section 39 of the mouth of beaker 12.

In this solution, in order to guarantee stability of the elastic insertion, on the external surface of the terminal section 39 of the mouth of beaker 12, there is a continuously formed circular tooth 45 which is coupled under pressure with the internal surface of the throat 138. This latter is substantially smooth, to facilitate the opening of the lid 14, when is latter has to be removed from the beaker 12.

In this, solution too the beaker 12 is provided with the circular fin 47 which guarantees hermetic tightness of the container 100 by means of coupling under pressure on the internal wall of the rim 37 of the lid 14.

## Claims

1. A container for picking up and preserving liquids sucked from patients and/or invalid people, comprising a beaker (12) and a lid (14) hermetically closed on the mouth of said beaker (12), wherein said lid (14) is provided, on one external surface thereof, with at least a first connecting element (16) communicating with the inside of said beaker (12) and suitable to be connected with a duct (19) for suction of said liquids, and with at least a second connecting element (17) communicating with the inside of said beaker (12) and suitable to be functionally connected to a vacuum source, wherein on the internal surface of said lid (14) a peripheral seat (38) is formed for elastic insertion of a corresponding terminal section (39) of said mouth of the beaker (12), and that mutual coupling means (45, 46) are provided to fasten said terminal section (39) to said peripheral seat (38), said peripheral seat comprising a circular throat (38) which is formed inside a substantially vertical rim (37), projecting from the internal surface of the lid (14), in such a way that said terminal section (39), being enclosed in the rim (37), is placed externally of the internal surface of the lid (14), **characterised in that** said mutual coupling means comprise first tooth means (45) formed on an external surface of said terminal section (39) and at least second tooth means (46) formed on an internal surface of said peripheral seat (38), said second tooth means (46) being angularly staggered with each other on the internal surface of said peripheral seat (38), the lid being made of plastic material, in correspondence of each tooth (46), the lid (14) having a slit (41) to further facilitate elastic spring-back of the plastic material.

2. A container according to claim 1, **characterised in that**, on the external surface of said beaker (12), there is a projection (47) which delimits said terminal section (39) and closes said peripheral seat (38) when in use.

3. A container according to claim 2, **characterised in that** said projection comprises a substantially horizontal circular fin (47) which co-operates, by beating, with said rim (37).

4. A container according to claim 1, **characterised in that** said first tooth means (45) are continuously formed on the entire surface of said terminal section (39).

5. A container according to any of the preceding claims, **characterised in that** it comprises a ring element (50) wherein said beaker (12) is housed and which acts as a wall-flxing element of the beaker (12) itself.

6. A container according to claim 5, **characterised in that** said ring element (50) can be removed from said beaker (12).

7. A container according to claim 6, **characterised in that** said ring element (50) comprises retaining means (52) suitable to hold a section of said suction duct (19) near said container (10).

8. A container according to any of the preceding claims, **characterised in that** said beaker (12) and said lid (14) are manufactured by moulding plastic material.

9. A container according to any of the preceding claims, **characterised in that** said lid (14) comprises a third connecting element (60) to connect the container (10) in series with other equal containers (10).

10. A container according to claim 1, wherein inside said beaker (12) a soft bag (115) is housed which is fixed on the internal surface of said lid (14).

## Patentansprüche

1. Behälter zum Aufnehmen und Aufbewahren von Flüssigkeiten, die von Patienten und/oder invaliden Personen abgesaugt werden, umfassend einen Becher (12) und einen Deckel (14), der die Öffnung des Bechers (12) hermetisch dicht verschließt, wobei der Deckel (14) an einer Außenfläche von ihm mit mindestens einem ersten Verbindungselement (16) versehen ist, das mit dem Inneren des Bechers (12) in Verbindung steht und dazu geeignet ist, mit einer Rohrleitung (19) zum Absaugen der Flüssigkeiten verbunden zu werden, und mit mindestens einem zweiten Verbindungselement (17), das mit dem Inneren des Bechers (12) in Verbindung steht und dazu geeignet ist, funktionsmäßig an eine Vakuumquelle angeschlossen zu werden, wobei an der Innenfläche des Deckels (14) eine Umfangsaufnahme (38) zum elastischen Einsetzen eines entsprechenden Endabschnitts (39) der Öffnung des Bechers (12) ausgebildet ist, und dass ineinander greifende Kopplungsmittel (45, 46) vorgesehen sind, um den Endabschnitt (39) an der Umfangsaufnahme (38) zu befestigen, wobei die Umfangsaufnahme eine kreisförmige Kehle (38) aufweist, die innerhalb eines im Wesentlichen vertikalen Randes (37) ausgebildet ist, und zwar von der Innenfläche des Deckels (14) vorstehend, derart, dass der im Rand (37) umschlossene Endabschnitt (39) sich außerhalb der Innenfläche des Deckels (14) befindet, **dadurch gekennzeichnet, dass** die ineinander greifenden Kopplungsmittel erste Verzahnungsmittel (45) umfassen, die an einer Außenfläche des Endabschnitts (39) ausgebildet sind, und mindestens zweite Verzahnungsmittel (46), die an einer Innenfläche der Umfangsaufnahme (38) ausgebildet sind, wobei die zweiten Verzahnungsmittel (46) an der Innenfläche der Umfangsaufnahme (38) winkelmäßig zueinander versetzt sind, der Deckel aus Kunststoffmaterial hergestellt ist, und der Deckel (14) in Entsprechung jedes Verzahnungsmittels (46) eine Ausnehmung (41) aufweist, um eine elastische Rückstellung des Kunststoffmaterials weiter zu erleichtern.

2. Behälter nach Anspruch 1, **dadurch gekennzeichnet, dass** auf der Außenfläche des Bechers (12) ein Vorsprung (47) vorhanden ist, der den Endabschnitt (39) begrenzt und im Gebrauch die Umfangsaufnahme (38) verschließt.

3. Behälter nach Anspruch 2, **dadurch gekennzeichnet, dass** der Vorsprung einen im Wesentlichen horizontalen, kreisförmigen Flansch (47) umfasst, der durch Schlagen mit dem Rand (37) zusammenwirkt.

4. Behälter nach Anspruch 1, **dadurch gekennzeichnet, dass** die ersten Verzahnungsmittel (45) durchgehend auf der gesamten Oberfläche des Endabschnitts (39) ausgebildet sind.

5. Behälter nach einem der vorhergehenden Ansprüche, **dadurch gekennzeichnet, dass** er ein Ringelement (50) aufweist, in welchem der Becher (12) aufgenommen ist und das als Wandfixierungselement des Bechers (12) selbst wirkt.

6. Behälter nach Anspruch 5, **dadurch gekennzeichnet, dass** das Ringelement (50) von dem Becher (12) abgenommen werden kann.

7. Behälter nach Anspruch 6, **dadurch gekennzeichnet, dass** das Ringelement (50) ein Haltemittel (52) aufweist, das dazu geeignet ist, einen Abschnitt der Rohrleitung (19) nahe am Behälter (10) zu halten.

8. Behälter nach einem der vorhergehenden Ansprüche, **dadurch gekennzeichnet, dass** der Becher (12) und der Deckel (14) durch Formen von Kunststoffmaterial gebildet sind.

9. Behälter nach einem der vorhergehenden Ansprüche, **dadurch gekennzeichnet, dass** der Deckel (14) ein drittes Verbindungselement (60) aufweist, um den Behälter (10) in Hintereinanderschaltung mit anderen, gleichartigen Behältern (10) zu verbinden.

10. Behälter nach Anspruch 1, wobei im Inneren des Bechers (12) ein weicher Beutel (115) aufgenommen ist, der an der Innenfläche des Deckel (14) fixiert ist.

## Revendications

1. Récipient destiné à prélever et à conserver des liquides aspirés provenant de patients et/ou de personnes invalides, comprenant un bécher (12) et un couvercle (14) fermé de manière étanche sur l'embouchure dudit bécher (12), dans lequel ledit couvercle (14) est muni, sur une surface externe de celui-ci, d'au moins un premier élément de raccord (16) en communication avec l'intérieur dudit bécher (12) et approprié pour être relié à un conduit (19) pour l'aspiration desdits liquides, et d'au moins un deuxième élément de raccord (17) en communication avec l'intérieur dudit bécher (12) et approprié pour être relié de manière fonctionnelle à une source d'aspiration, dans lequel, sur la surface interne dudit couvercle (14), un siège périphérique (38) est formé pour l'insertion élastique d'une section terminale correspondante (39) de ladite embouchure du bécher (12) et des moyens de couplage mutuel (45, 46) sont fournis pour fixer ladite section terminale (39) audit siège périphérique (38), ledit siège périphérique comprenant une gorge circulaire (38) qui est formée à l'intérieur d'un rebord sensiblement vertical (37), faisant saillie à partir de la surface interne du couvercle (14), de telle sorte que ladite section terminale (39), étant enfermée dans le rebord (37), est placée de manière externe à la surface interne du couvercle (14), **caractérisé en ce que** lesdits moyens de couplage mutuel comprennent des premiers moyens dentés (45) formés sur une surface externe de ladite section terminale (39) et au moins des deuxièmes moyens dentés (46) formés sur une surface interne de dudit siège périphérique (38), lesdits deuxièmes moyens dentés (46) étant décalés de manière angulaire les uns par apport aux autres sur la surface interne dudit siège périphérique (38), le couvercle étant fabriqué dans une matière plastique, en face de chaque dent (46), le couvercle (14) présentant une fente (41) pour faciliter davantage le retour élastique de la matière plastique.

2. Récipient selon la revendication 1, **caractérisé en ce que**, sur la surface externe dudit bécher (12), une saillie (47) délimite ladite section terminale (39) et ferme ledit siège périphérique (38) lors de l'utilisation.

3. Récipient selon la revendication 2, **caractérisé en ce que** ladite saillie comprend une ailette circulaire sensiblement horizontale (47) qui coopère, par enfoncement, avec ledit rebord (37).

4. Récipient selon la revendication 1, **caractérisé en ce que** lesdits premiers moyens dentés (45) sont formés de manière continue sur la surface totale de ladite section terminale (39).

5. Récipient selon l'une quelconque des revendications précédentes, **caractérisé en ce qu'**il comprend un élément circulaire (50) dans lequel ledit bécher (12) est logé et qui agit comme un élément de fixation de paroi du bécher (12) lui-même.

6. Récipient selon la revendication 5, **caractérisé en ce que** ledit élément circulaire (50) peut être retiré dudit bécher (12).

7. Récipient selon la revendication 6, **caractérisé en ce que** ledit élément circulaire (50) comprend des moyens de retenue (52) appropriés pour maintenir une section dudit conduit d'aspiration (19) à proximité dudit récipient (10).

8. Récipient selon l'une quelconque des revendications précédentes, **caractérisé en ce que** ledit bécher (12) et ledit couvercle (14) sont fabriqués par moulage de matière plastique.

9. Récipient selon l'une quelconque des revendications précédentes **caractérisé en ce que** ledit couvercle (14) comprend un troisième élément de raccord (60) pour relier le récipient (10) en série à d'autres récipients équivalents (10).

10. Récipient selon la revendication 1, dans lequel, à l'intérieur dudit bécher (12) est logée une poche souple (115) qui est fixée sur la surface interne dudit couvercle (14).
